# DEMANDE DE BREVET EUROPEEN

(11) **EP 0 966 945 A1**
(43) Date de publication de la demande: **29.12.1999**
(21) Numéro de dépôt: 99401295.3
(22) Date de dépôt: 31.05.1999
(51) Int. Cl.: A61K 7/06

(54) **Compositions cosmétiques détergentes et utilisation**

(30) Priorité: 16.06.1998 FR 9807583
(71) Demandeur: L'OREAL, 75008 Paris (FR)
(72) Inventeur: Decoster, Sandrine, 93800 Epinay/Seine (FR); Beauquey, Bernard, 92110 Clichy (FR)
(74) Mandataire: Le Blainvaux Bellegarde, Françoise

(57) **Abrégé**

L'invention concerne de nouvelles compositions détergentes et conditionnantes comprenant, dans un milieu cosmétiquement acceptable, (A) une base lavante et (B) au moins une silicone comprenant au moins un groupement glycérol.

Application au nettoyage et au soin des cheveux ou de la peau.

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, une base lavante constituée de tensioactifs à pouvoir détergent dans laquelle est également présent à titre d'agents conditionneurs une silicone soluble dans l'eau à groupement glycérol. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Dans ce but, on a déjà proposé d'utiliser des silicones et plus particulièrement des silicones insolubles. En effet, jusqu'à présent, pour avoir un dépôt de silicone sur les matières kératiniques lors du rinçage, on a utilisé des silicones insolubles. Les composés insolubles et plus particulièrement les silicones présentent l'inconvénient d'être difficiles à maintenir en dispersion régulière dans le milieu.

Les silicones doivent également être véhiculées sur les matières kératiniques traitées en vue de leur conférer, suivant l'application, des propriétés de douceur, de brillance et de démêlage sans induire de caractère gras.

Ainsi, à la suite d'importantes recherches menées sur la question, il a maintenant été trouvé par la Demanderesse, qu'en utilisant une base lavante et au moins une silicone contenant au moins un groupement glycérol soluble dans la composition, il est possible d'obtenir des compositions détergentes stables présentant d'excellentes propriétés cosmétiques, en particulier la facilité de coiffage, le maintien, la nervosité et le volume des cheveux traités et ayant de bonnes propriétés d'usage tel qu'un bon pouvoir lavant intrinsèque et un bon pouvoir moussant.

La mise en oeuvre industrielle est extrêmement facile et les propriétés cosmétiques des shampooings sont nettement supérieures à celles des shampooings contenant des silicones solubles telles que les silicones à groupement(s) oxyéthyléné(s).

Les compositions conformes à l'invention confèrent aux cheveux, après rinçage, un remarquable effet traitant qui se manifeste notamment par une facilité de démêlage, ainsi qu'un apport de volume, de légèreté, de lissage, de douceur et de souplesse sans aucune sensation de gras.

Ainsi, la présente invention a pour objet de nouvelles compositions cosmétiques détergentes et conditionnantes caractérisée par le fait qu'elles comprennent, dans un milieu aqueux cosmétiquement acceptable, (A) une base lavante et (B) au moins une silicone comprenant au moins un groupement glycérol et ne comprenant pas de fonction ester carboxylique.

L'invention a également pour objet l'utilisation en cosmétique des compositions ci-dessus pour le nettoyage et /ou le démaquillage et/ou le conditionnement des matières kératiniques telles que les cheveux et la peau.

### A- BASE LAVANTE :

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotères, non ioniques et cationiques.

Toutefois, selon l'invention, la base lavante comprend de préférence des tensioactifs anioniques ou des mélanges de tensioactifs anioniques et de tensioactifs amphotères ou de tensioactifs non ioniques.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL®, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R_{2'}-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂, désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHONE POULENC.

Dans les compositions conformes à l'invention, on utilise de préférence des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL® C2M CONC" en solution aqueuse à 38 % de matière active ou sous la dénomination MIRANOL® C32;
- soit un agent tensioactif amphotère de type zwittérionique tel que les alkylbétaïnes en particulier la cocoylbétaïne commercialisée sous la dénomination "DEHYTON® AB 30" en solution aqueuse à 32 % de MA par la société HENKEL ou les alkylamidobétaïnes telles que la TEGOBETAINE® F50 commercialisée par la société GLODSCHMIDT.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique. On notera que les tensioactifs cationiques, dont l'utilisation n'est pas exclue, ne constituent pas des tensioactifs préférés pour la mise en oeuvre de la présente invention.

### B- SILICONE GLYCEROL

Les compositions selon l'invention comprennent nécessairement une silicone comprenant au moins un groupement glycérol, soluble dans la composition et ne comprenant pas de fonction ester.

Les silicones comprenant au moins un groupement glycérol sont par exemple choisies parmi les composés de formules générales (I) et (II) : 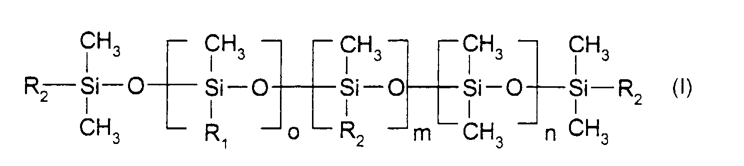 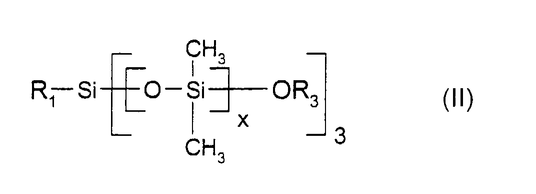 formules dans lesquelles :
R₁, identique ou différent, représente un radical alkyle, linéaire ou ramifié, saturé ou insaturé, en C₁-C₃₀ ou phényle.
R₂, identique ou différent, représente un radical choisi parmi :
R₁, 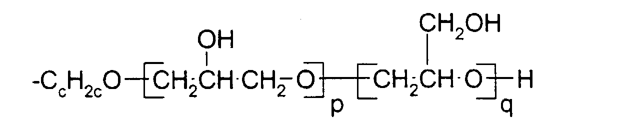 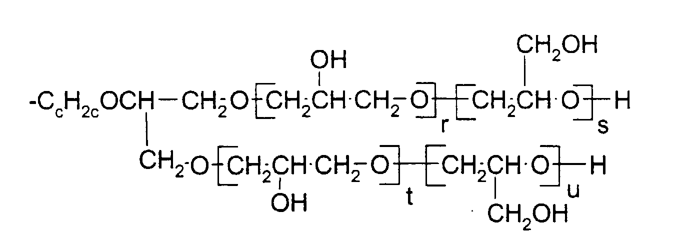
R₃, identique ou différent, représente un radical choisi parmi : 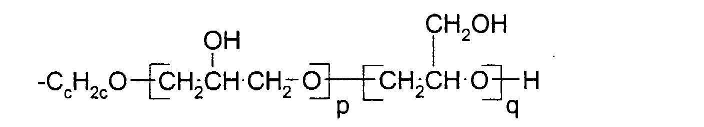 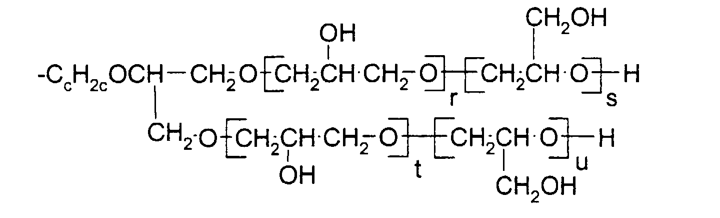
   - c peut varier de 1 à 10,
   - m peut varier de 1 à 50,
   - n peut varier de 0 à 500,
   - o peut varier de 0 à 20,
   - p peut varier de 0 à 50,
   - q peut varier de 0 à 50,
   - p + q est supérieur ou égal à 1 et est de préférence inférieur à 100,
   - r peut varier de 0 à 50,
   - s peut varier de 0 à 50,
   - r + s est supérieur ou égal à 1 et est de préférence inférieur à 100,
   - t peut varier de 0 à 50,
   - u peut varier de 0 à 50,
   - t + u est supérieur ou égal à 1 et est de préférence inférieur à 100,
   - x peut varier de 1 à 100,
sous réserve qu'au moins un radical R₂ ne désigne pas R_{1.}

Plus particulièrement, ces formules répondent à au moins une des, et de préférence toutes les, conditions suivantes :
- R₁ désigne le radical méthyle.
- R₂désigne : 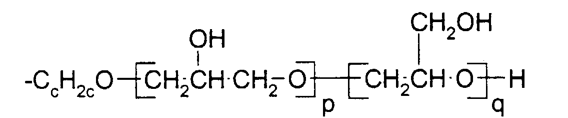
- c est égal à 2 ou 3.
- q varie de 0 à 20, de préférence de 0 à 10,
- p varie de 0 à 20, de préférence de 0 à 10.
- p + q varie de 1 à 10

De préférence, on utilise les silicones glycérol répondant à la formule générale (I).

Les silicones selon l'invention sont par exemple des polydiméthylsiloxane ayant au moins un groupement de formule (A) ou (B)

-CH₂-CH₂-CH₂-O-(CH₂-CH(CH₂OH)-O)ₚ-H (A)

-CH₂-CH₂-CH₂-O-CH₂-CH(OH)-CH₂-O-(CH₂-CH(CH₂OH)-O)_{q}-H (B)

dans lesquelles p et q sont des nombres, de préférence compris entre 1 et 50, plus particulièrement entre 1 et 10.

De tels silicones sont notamment décrites dans le brevet US4 431 789 et la demande JP09 071504.

Des produits convenant particulièrement bien pour la réalisation de l'invention sont par exemple les polydiméthylsiloxanes à groupements triglycérol proposés par SHIN-ETSU sous les dénominations X22-4947, X22-6147 et X22-6148.

La ou les silicones peuvent être utilisées dans les compositions conformes à l'invention dans des concentrations généralement comprises entre 0,1 et 15 %, et de préférence entre 0,2 et 10 % en poids par rapport au poids total de la composition et encore plus particulièrement de 0,5 à 5% en poids.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les alcools gras supérieurs à C16, les palmitates de sodium ou de magnésium, les stéarates et hydroxystéarates de sodium ou de magnésium, les alcool gras, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensioactifs cationiques, les polymères anioniques ou non ioniques ou cationiques ou amphotères, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les silicones différentes des silicones de l'invention, volatiles ou non volatiles, solubles et insolubles dans le milieu et leurs mélanges.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », «MERQUAT 550» et «MERQUAT S» par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association (base lavante + silicone glycérol) conforme à l'invention ne soient pas, ou substantiellement pas, altérées par la ou les adjonctions envisagées.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés.

### EXEMPLE

On a réalisé deux compositions de shampooings, l'une conforme à l'invention (composition A) et l'autre comparative (composition B) :

| | A Invention | B Comparatif |
|---|---|---|
| - Lauryléthersulfate de sodium (C12/C14 à 70/30) à 2,2 moles d'oxyde d'éthylène en solution aqueuse à 28% de MA (MA = matière active) | 15 gMA | 15 gMA |
| - Cocoylbétaïne (DEHYTON AB 30) | 2,7 gMA | 2,7 gMA |
| - Silicone triglycérol (*) | 2,7 g | - |
| - Silicone oxyéthyléné(**) | - | 2,7 g |
| Gomme de guar cationique | 0,05 g | 0,05 g |
| - Mélange d'alcool cétylique et de 1-(hexadécyloxy)-2-octadécanol | 2,5 g | 2,5 g |
| - Monoisopropanolamide d'acides de coprah | 1 g | 1 g |
| - Parfum, conservateur | qs | qs |
| - Acide citrique qs pH | 7,6 | 7,6 |
| - Eau déminéralisée qs | 100 g | 100 g |

| | | |
|---|---|---|
| (*)Silicone triglycérol : Polydiméthylsiloxane à groupements triglycérol commercilisé par la société SHIN-ETSU sous la dénomination X22 4947. | | |
| (**) Silicone oxyéthylénée : Polydiméthylsiloxane à groupements oxyéthyléné commercialisé par la société DOW CORNING sous la dénomination DC193. | | |

On a effectué un shampooing en appliquant environ 12 g de la composition A sur des cheveux sensibilisés préalablement mouillés. On fait mousser le shampooing puis on rince abondamment à l'eau.
On procède selon le même mode opératoire que ci-dessus avec la composition comparative B.

Un panel d'experts a évalué l'aspect des cheveux.

Tous les experts indiquent que les cheveux traités avec la composition A selon l'invention sont plus doux et plus lisses que les cheveux traités avec la composition B.

## Revendications

1. Composition cosmétique détergente et conditionnante, caractérisée par le fait qu'elle comprend, dans un milieu aqueux cosmétiquement acceptable, (A) une base lavante et (B) au moins une silicone ayant au moins un groupement glycérol et ne comprenant pas de fonction ester.

2. Composition selon la revendication 1, caractérisée par le fait que ladite base lavante comprend un ou plusieurs tensioactifs choisis parmi des tensioactifs anioniques, amphotères, non ioniques, cationiques et leurs mélanges.

3. Composition selon la revendication 1 ou 2, caractérisée par le fait que ladite base lavante est présente à une teneur pondérale comprise entre 4 % et 50 % en poids par rapport au poids total de la composition, de préférence comprise entre 6 % et 35 % en poids et plus préférentiellement comprise entre 8 % et 25 % en poids.

4. Composition selon l'une quelconque des revendications 1 à 3, caractérisée en ce que la silicone ayant au moins un groupement glycérol est choisie parmi les composés de formules générales (I) et (II) : 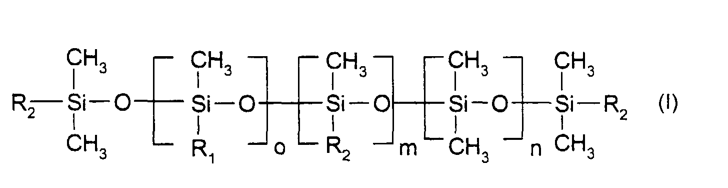 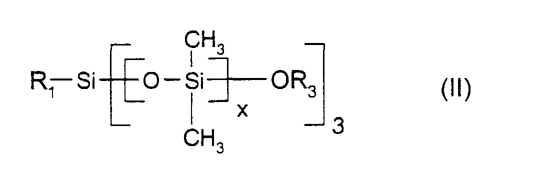 formules dans lesquelles :
R₁, identique ou différent, représente un radical alkyle, linéaire ou ramifié, saturé ou insaturé, en C₁-C₃₀ ou phényle.
R₂, identique ou différent, représente un radical choisi parmi :
R₁, 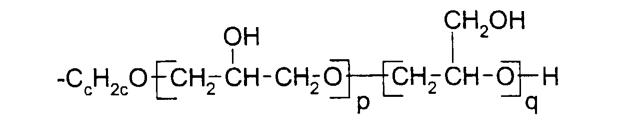 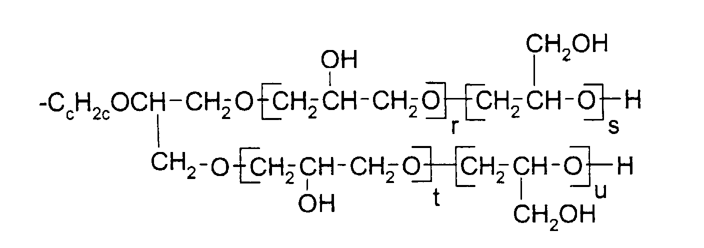
R₃, identique ou différent, représente un radical choisi parmi : 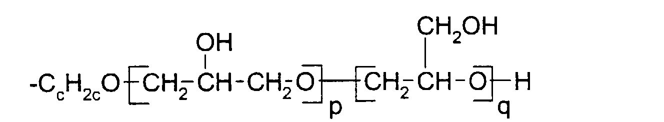 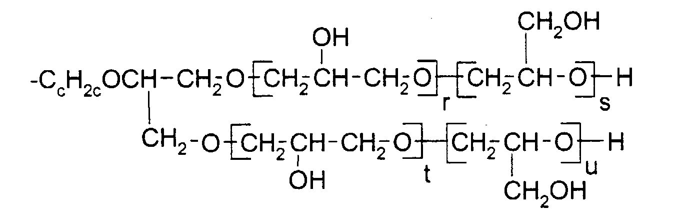
- c varie de 1 à 10,
- m varie de 1 à 50,
- n varie de 0 à 500,
- o varie de 0 à 20,
- p varie de 0 à 50,
- q varie de 0 à 50,
- p + q est supérieur ou égal à 1,
- r varie de 0 à 50,
- s varie de 0 à 50,
- r + s est supérieur ou égal à 1,
- t varie de 0 à 50,
- u varie de 0 à 50,
- t + u est supérieur ou égal à 1,
- x varie de 1 à 100,
sous réserve qu'au moins un radical R₂ ne désigne pas R_{1.}

5. Composition selon la revendication 4, caractérisée en ce que la silicone de formules (I) ou (II) répond à au moins une des, et de préférence toutes les, conditions suivantes :
- R₁ désigne le radical méthyle.
- R₂ désigne : 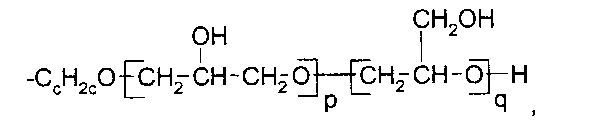
- c est égal à 2 ou 3.
- q varie de 0 à 20, de préférence de 0 à 10,
- p varie de 0 à 20, de préférence de 0 à 10.
- p + q varie de 1 à 10

6. Composition selon l'une quelconque des revendications 4 ou 5, caractérisée en ce que la silicone ayant au moins un groupement glycérol est un composé de formule générale (I).

7. Composition selon l'une quelconque des revendications 1 à 6, caractérisée en ce que la silicone ayant au moins un groupement glycérol est choisie parmi les polydiméthylsiloxanes ayant au moins un groupement de formule (A) ou (B)
-CH₂-CH₂-CH₂-O-(CH₂-CH(CH₂OH)-O)ₚ-H (A)
-CH₂-CH₂-CH₂-O-CH₂-CH(OH)-CH₂-O-(CH₂-CH(CH₂OH)-O)_{q}-H (B)
dans lesquelles p et q sont des nombres compris entre 1 et 10.

8. Composition selon l'une quelconque des revendications 1 à 7, caractérisée par le fait que la silicone est présente dans des concentrations comprises entre 0,1 et 15 %, et de préférence entre 0,2 et 10 % en poids par rapport au poids total de la composition.

9. Composition selon l'une quelconque des revendications 1 à 8, caractérisée par le fait qu'elle contient en plus un ou plusieurs adjuvants destinés à améliorer les propriétés cosmétiques choisis par les tensioactifs cationiques, les polymères anioniques ou non ioniques ou cationiques ou amphotères, les protéines, les céramides, les pseudocéramides ou les silicones.

10. Composition selon la revendication 9, caractérisée par le fait le polymère cationique est choisi parmi les dérivés d'éther de cellulose quaternaires, les homopolymères de sel de diallyldiméthylammonium et les copolymères de sel de diallyldiméthylammonium et d'acrylamide, les polysaccharides cationiques.

11. Composition selon la revendication 10 caractérisée en ce que le polymère cationique représente de 0,001 % à 10 % en poids, de préférence de 0,005 % à 5 % en poids, et encore plus préférentiellement de 0,01 % à 3 % en poids, du poids total de la composition.

12. Utilisation d'une composition telle définie dans l'une quelconque des revendications 1 à 11 pour le nettoyage et/ou le démaquillage et/ou le conditionnement des matières kératiniques.

13. Procédé de lavage et de conditionnement des matières kératiniques telles que les cheveux consistant à appliquer sur lesdites matières mouillées une quantité efficace d'une composition telle que définie dans l'une quelconque des revendications 1 à 11, puis à effectuer un rinçage à l'eau après un éventuel temps de pause.
